# EUROPEAN PATENT APPLICATION

(11) **EP 4 510 140 A1**
(43) Date of publication of application: **19.02.2025**
(21) Application number: 24192132.9
(22) Date of filing: 31.07.2024
(51) Int. Cl.: G16H 10/60, G16H 30/40

(54) **APPARATUS AND METHOD FOR ANONYMIZING MEDICAL IMAGES**

(30) Priority: 31.07.2023 KR 20230099656
(71) Applicant: Coreline Soft Co., Ltd, Seoul 03991 (KR)
(72) Inventor: YI, Jaeyoun, 10307 Goyang-si, Gyeonggi-do (KR); YU, Donghoon, 10073 Gimpo-si, Gyeonggi-do (KR); SEO, Hyungi, 02831 Seoul (KR)
(74) Representative: Stöckeler, Ferdinand

(57) **Abstract**

Disclosed are a method of anonymizing medical images, the method comprising: acquiring or receiving a first medical image; separating an identification area containing anatomical structure information and a de-identification area containing a skin area in the first medical image; generating a de-identified image including a plurality of pattern areas with a predetermined size for the de-identification area; and generating a second medical image by replacing the de-identification area with the de-identified image.

## Description

### CROSS-REFERENCE TO RELATED APPLICATION

This application claims under 35 U.S.C. §119(a) the benefit of Korean Patent Application No. 10-2023-0099656 filed on July 31, 2023, which is incorporated herein by reference.

### TECHNICAL FIELD

The present invention relates to technology for processing, analyzing, and visualizing medical images, and more particularly to technology for de-identifying an area other than an area requiring diagnosis while preserving anatomical structure information.

### BACKGROUND ART

The contents described in this section merely provide information about the related art of the present invention and do not constitute prior art.

Recently, health care big-data analysis technology using medical information (or health information) has attracted attention. To implement this healthcare big-data analysis technology, large amounts of medical information must first be collected. However, in connection with the collection of medical information, issues regarding violation of laws related to the protection of personal information are emerging.

Accordingly, there have been proposed various pseudonymization technologies that de-identify personal information by pseudonymizing the personal information in medical information. In this case, pseudonymization refers to the process of assigning a random code or number (a pseudonym) to personal information, making it impossible to identify a specific individual without the use and combination of additional information required to restore the personal information to its original state. Furthermore, the information that has been pseudonymized in this manner is referred to as "pseudonymized information."

The prior art pseudonymization technologies for medical information are merely pseudonymization technologies for personal information in the form of text related to personal information such as a name, age, gender, a phone number, and/or the like. In addition to these types of personal information in the form of text, medical images in the form of images may also be recognized as personal information in some cases. However, so far, there has been no pseudonymization technology for medical images, i.e., technology for generating pseudonymized images, which are pseudonymized information, by pseudonymizing medical images.

Meanwhile, in order to pseudonymize medical images, there may be proposed technology that applies a simple image processing technique, such as masking or blurring, to specific areas in medical images. However, in the case of this prior art, there is a problem in that the reality of a pseudonymized image itself obtained as a result of image processing is low. For example, when a medical image of a human face is pseudonymized using the prior art technology, a generated pseudonymized image may be recognized as not being an image of a human face by medical personnel (a doctor and/or the like), or may be significantly altered and recognized as an image of the face of a person who does not actually exist.

### SUMMARY OF THE DISCLOSURE

In the above-described prior arts, there is disclosed a configuration in which a part requiring diagnosis and a part not requiring diagnosis in a medical image are separated as an identification area and a de-identification area, respectively, and a de-identified image is generated by editing or processing the de-identification area.

However, these prior arts directly edit finally generated facial images, coronal images, sagittal images, etc., so that overall diagnostic information for areas masked by de-identification areas is lost.

Accordingly, the anonymization/pseudonymization method of the prior arts can generate only a one-time de-identified image, and anatomical information contained in an original medical image is lost for a de-identification area and cannot be utilized.

The anonymization/pseudonymization method of the prior arts cannot provide incidental findings based on anatomical information contained in an original medical image.

The present invention has been conceived to overcome the problems of the prior arts, and an object of the present invention is to propose technology that efficiently preserves anatomical information contained in an original medical image during the process of anonymizing/pseudonymizing the medical image.

An object of the present invention is to propose anonymization/pseudonymization technology that may provide incidental findings based on anatomical information contained in an original medical image.

An object of the present invention is to propose anonymization/pseudonymization technology that prevents personal information from being exposed by increasing the level of de-identification of medical images.

According to an aspect of the present invention, there may be provided a method of anonymizing medical images, the method comprising: acquiring or receiving a first medical image; separating an identification area containing anatomical structure information and a de-identification area containing a skin area in the first medical image; generating a de-identified image including a plurality of pattern areas with a predetermined size for the de-identification area; and generating a second medical image by replacing the de-identification area with the de-identified image.

In the method of the embodiment of the present invention, the plurality of pattern areas may comprise first pattern areas having a first signal intensity value and second pattern areas having a second signal intensity value.

In the method of the embodiment of the present invention, each of the plurality of pattern areas may be generated to have a randomized signal intensity value.

In the method of the embodiment of the present invention, each of the plurality of pattern areas may have a signal intensity value generated based on a representative value of signal intensity values of a de-identification area in each of the plurality of pattern areas.

In the method of the embodiment of the present invention, the generating the de-identified image may comprise: determining a second de-identification area including at least a portion of the de-identification area and having a thickness based on a user input; and generating the plurality of pattern areas having a predetermined size by using a representative value of signal intensity values in the second de-identification area.

In the method of the embodiment of the present invention, the first medical image may be a magnetic resonance (MR) image set or a computed tomography (CT) image set including a plurality of slices; and the separating, the generating the de-identified image, and the generating the second medical image may be performed for each of the plurality of slices in the CT image set or the MR image set.

In the method of the embodiment of the present invention, the identification area may comprise information that can restore a three-dimensional (3D) anatomical structure based on the anatomical structure information in the first medical image.

According to an aspect of the present invention, there may be provided an apparatus for anonymizing medical images, the apparatus comprising: a memory configured to store one or more instructions; and a processor configured to execute the one or more instructions.

In the apparatus of the embodiment of the present invention, the processor, by executing the one or more instructions, may be configured to: acquire or receives a first medical image; separate an identification area containing anatomical structure information and a de-identification area containing a skin area in the first medical image; generate a de-identified image including a plurality of pattern areas with a predetermined size for the de-identification area; and generate a second medical image by replacing the de-identification area with the de-identified image.

In the apparatus of the embodiment of the present invention, the plurality of pattern areas may comprise first pattern areas having a first signal intensity value and second pattern areas having a second signal intensity value.

In the apparatus of the embodiment of the present invention, each of the plurality of pattern areas may be generated to have a randomized signal intensity value.

In the apparatus of the embodiment of the present invention, each of the plurality of pattern areas may have a signal intensity value generated based on a representative value of signal intensity values of a de-identification area in each of the plurality of pattern areas.

In the apparatus of the embodiment of the present invention, the processor, by executing the one or more instructions, may be further configured to: determine a second de-identification area including at least a portion of the de-identification area and having a thickness based on a user input; and generate the plurality of pattern areas having a predetermined size by using a representative value of signal intensity values in the second de-identification area.

In the apparatus of the embodiment of the present invention, the first medical image may be a magnetic resonance (MR) image set or a computed tomography (CT) image set including a plurality of slices.

In the apparatus of the embodiment of the present invention, the processor, by executing the one or more instructions, may be further configured to separate the identification area and the de-identification area, generate the de-identified image for the de-identification area and generate the second medical image for each of the plurality of slices in the CT image set or the MR image set.

In the apparatus of the embodiment of the present invention, the identification area may comprise information that can restore a three-dimensional (3D) anatomical structure based on the anatomical structure information in the first medical image.

According to an embodiment of the present invention, anatomical information contained in an original medical image may be efficiently preserved during the process of anonymizing/pseudonymizing the medical image.

According to an embodiment of the present invention, there may be provided anonymization/pseudonymization technology that can provide incidental findings based on anatomical information contained in an original medical image.

The present invention may provide anonymization/pseudonymization technology that prevents personal information from being exposed by increasing the level of de-identification of medical images.

### BRIEF DESCRIPTION OF THE DRAWINGS

The above and other objects, features and advantages of the present invention will be more clearly understood from the following detailed description taken in conjunction with the accompanying drawings, in which:
FIG. 1 is an operational flowchart showing a method of anonymizing medical images according to an embodiment of the present invention;
FIG. 2 is an operational flowchart showing an embodiment of step S300 shown in FIG. 1;
FIG. 3 is a conceptual diagram showing a process of anonymizing medical images according to an embodiment of the present invention;
FIG. 4 is a conceptual diagram showing a process of anonymizing medical images according to an embodiment of the present invention;
FIG. 5 is a conceptual diagram showing a medical image diagnosis environment in which the results of anonymization/pseudonymization of medical images may be utilized according to an embodiment of the present invention;
FIG. 6 is a diagram showing 3D rendering results based on an original medical image;
FIG. 7 is a diagram showing the results of 3D rendering performed in the state in which a de-identification area having a thickness of 2 mm is generated;
FIG. 8 is a diagram showing the results of 3D rendering performed in the state in which a de-identification area having a thickness of 4 mm is generated;
FIG. 9 is a diagram showing the results of 3D rendering performed in the state in which a de-identification area having a thickness of 6 mm is generated;
FIG. 10 is a diagram showing the results of 3D rendering performed in the state in which a de-identified image having a 2 mm thickness is generated and then a de-identified image is generated using a mosaic technique;
FIG. 11 is a conceptual diagram showing part of a process of anonymizing/pseudonymizing medical images according to an embodiment of the present invention; and
FIG. 12 is a conceptual diagram showing an example of a generalized medical image processing, analysis, visualization, diagnostic assistance, and anonymization/pseudonymization apparatus or computing system capable of performing at least part of the process of FIGS. 1 to 11.

### DETAILED DESCRIPTION OF THE DISCLOSURE

Other objects and features of the present invention in addition to the above-described objects will be apparent from the following description of embodiments to be given with reference to the accompanying drawings.

The embodiments of the present invention will be described in detail below with reference to the accompanying drawings. In the following description, when it is determined that a detailed description of a known component or function may unnecessarily make the gist of the present invention obscure, it will be omitted.

Relational terms such as first, second, and the like may be used for describing various elements, but the elements should not be limited by the terms. These terms are only used to distinguish one element from another. For example, a first component may be named a second component without departing from the scope of the present disclosure, and the second component may also be similarly named the first component. The term "and/or" means any one or a combination of a plurality of related and described items.

When it is mentioned that a certain component is "coupled with" or "connected with" another component, it should be understood that the certain component is directly "coupled with" or "connected with" to the other component or a further component may be disposed therebetween. In contrast, when it is mentioned that a certain component is "directly coupled with" or "directly connected with" another component, it will be understood that a further component is not disposed therebetween.

The terms used in the present disclosure are only used to describe specific exemplary embodiments, and are not intended to limit the present disclosure. The singular expression includes the plural expression unless the context clearly dictates otherwise. In the present disclosure, terms such as 'comprise' or 'have' are intended to designate that a feature, number, step, operation, component, part, or combination thereof described in the specification exists, but it should be understood that the terms do not preclude existence or addition of one or more features, numbers, steps, operations, components, parts, or combinations thereof.

Unless otherwise defined, all terms (including technical and scientific terms) used herein have the same meaning as commonly understood by one of ordinary skill in the art to which this disclosure belongs. Terms that are generally used and have been in dictionaries should be construed as having meanings matched with contextual meanings in the art. In this description, unless defined clearly, terms are not necessarily construed as having formal meanings.

Meanwhile, even when the technology was known before the filing date of the present application, it may be included as part of the configuration of the present invention of the present application when necessary, and this will be described herein to the extent that it does not obscure the purpose of the present invention. However, in the following description of the configuration of the present invention of the present application, detailed descriptions of items that can be clearly understood by those skilled in the art as the technologies known before the filing date of the present application may obscure the purpose of the present invention, so that excessively detailed descriptions of known technologies will be omitted.

For example, technologies known before the application of the present invention may be used as technologies for generating pseudonymized/anonymized images by processing medical images using a mosaic technique, blurring, or the like, and at least some of these known technologies may be applied as elemental technologies required for implementing the present invention. For example, descriptions of elemental technologies required for implementing parts of the configuration of the present invention may be replaced by providing notification that the technologies are known to those skilled in the art through Japanese Patent Application Publication No. 2021-051471 entitled "Anonymization Apparatus and Anonymization System," Japanese Patent Application Publication No. 2021-186087 entitled "Medical Image Processing Apparatus, and Medical Image Processing Method and Program," and Korean Patent Application Publication No. 10-2022-0026845 entitled "Method and Apparatus for Pseudonymizing Medical Images."

However, the purpose of the present invention is not to claim rights to these known technologies, and the content of the known technologies may be included as part of the present invention within the range that does not depart from the spirit of the present invention.

Hereinafter, embodiments of the present invention will be described in more detail with reference to the accompanying drawings. In order to facilitate overall understanding when describing the present invention, the same reference numerals will be used for the same components in the drawings, and redundant descriptions of the same components will be omitted.

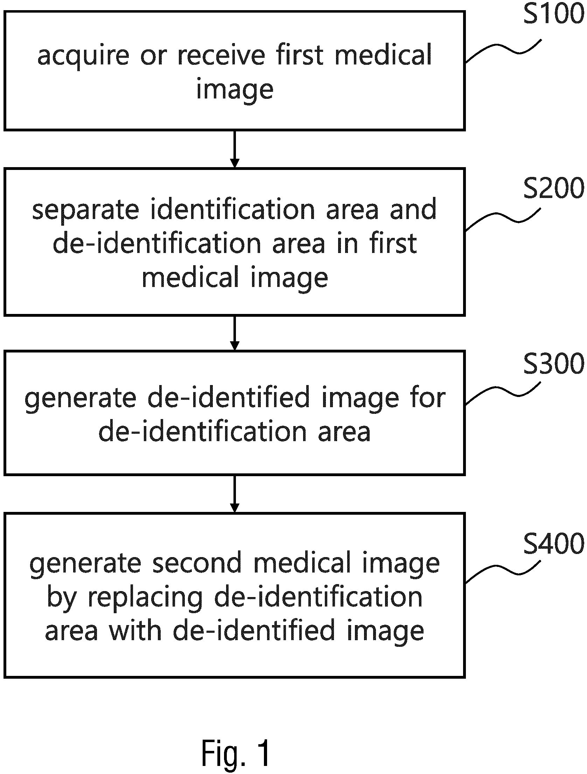

FIG. 1 is an operational flowchart showing a method of anonymizing medical images according to an embodiment of the present invention.

Referring to FIG. 1, the method of anonymizing medical images according to the present embodiment includes: step S100 of acquiring or receiving a first medical image; step S200 of separating an identification area containing anatomical structure information and a de-identification area containing a skin area in the first medical image; step S300 of generating a de-identified image including a plurality of pattern areas with a predetermined size by applying a mosaic technique to the de-identification area; and step S400 of generating a second medical image in which the surface of the human body is anonymized/pseudonymized by replacing the de-identification area with the de-identified image.

The segmentation of skin area may be utilized in the step of S200 of the separating the identification area and the de-identification area in the first medical image.

The first medical image may be a magnetic resonance (MR) image set or a computed tomography (CT) image set including a plurality of slices. Step S200 of separating an identification area and a de-identification area, step S300 of generating a de-identified image for the de-identification area, and step S400 of generating a second medical image may be performed for each of the plurality of slices in the CT image set or the MR image set.

The identification area may include information that can restore a three-dimensional (3D) anatomical structure based on anatomical structure information in the first medical image. Incidental findings may be diagnosed using the second medical image based on the information that can restore the 3D anatomical structure included in the identification area.

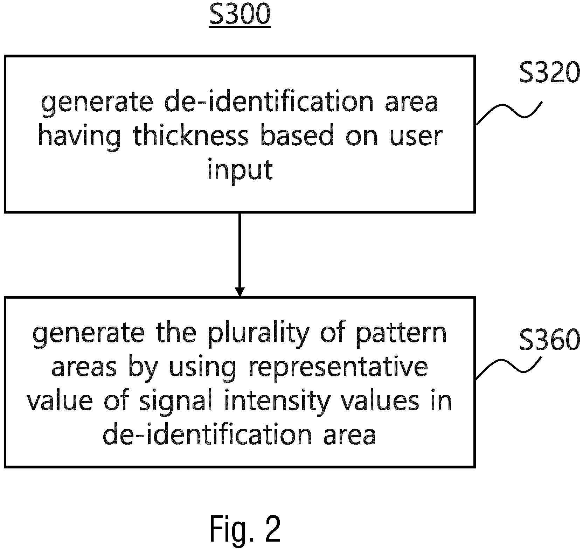

FIG. 2 is an operational flowchart showing an embodiment of step S300 shown in FIG. 1.

Step S300 of generating the de-identified image for the de-identification area may include step S320 of generating a second de-identification area including at least a portion of the de-identification area and having a thickness based on a user input. In the step S320, the second de-identification area may be newly determined, re-determined, and/or re-defined so as to have the thickness based on the user input.

Step S300 of generating the de-identified image for the de-identification area may include step S360 of generating the plurality of pattern areas having a predetermined size by using the representative value of signal intensity values in the second de-identification area.

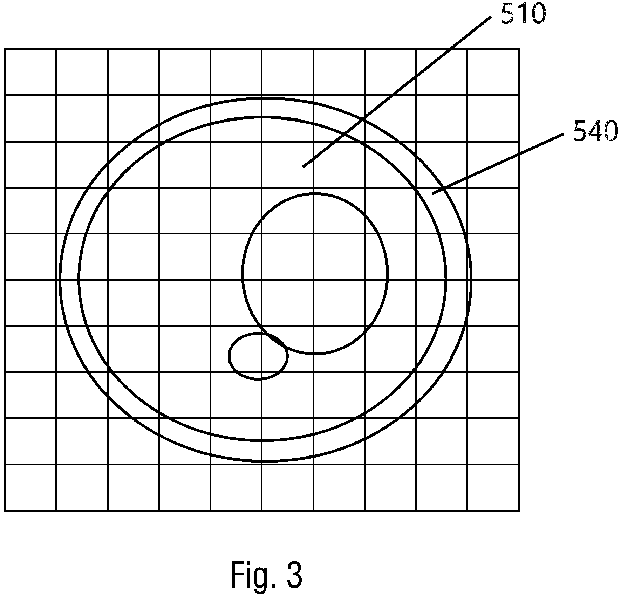

FIG. 3 is a conceptual diagram showing a process of anonymizing medical images according to an embodiment of the present invention.

Through step S200, a first identification area 510 including anatomical information and a de-identification area 540 may be separated from each other.

The predetermined thickness of the de-identification area 540 may be given in a predetermined manner, or may be determined by a user input. The thickness of the de-identification area 540 may be adjusted through step S320.

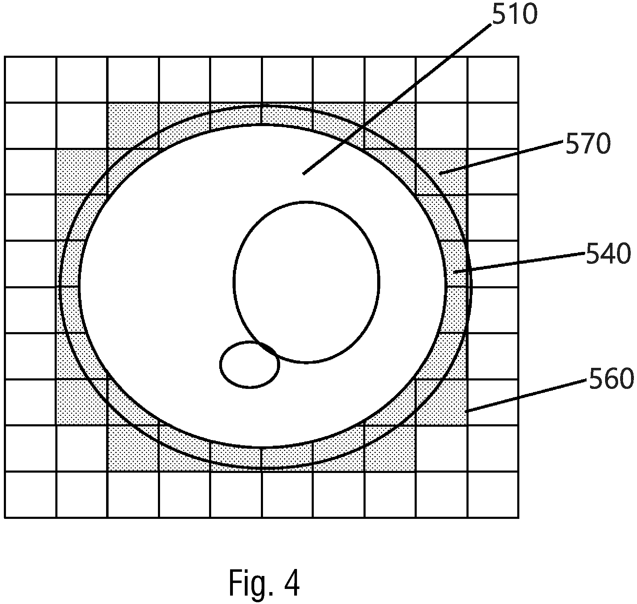

FIG. 4 is a conceptual diagram showing a process of anonymizing medical images according to an embodiment of the present invention.

Through step S360, a de-identified image 560 including the de-identification area 540 may be generated. The de-identified image 560 may include a plurality of pattern areas 570 including the de-identification area 540.

The plurality of pattern areas 570 may include first pattern areas having a first signal intensity value and second pattern areas having a second signal intensity value different from the first signal intensity value.

Although an embodiment in which the plurality of pattern areas 570 are in the form of square blocks and are anonymized using a mosaic technique is shown in FIG. 4, the spirit of the present invention is not limited to this embodiment. The pattern areas 570 may have various shapes.

The pattern areas 570 may be polygonal, such as triangles or squares, or may have various geometric shapes, such as circles, semicircles, or ellipses. Each of the pattern areas 570 may be anonymized to a typical signal intensity value despite the signal intensity value within the original medical image, so that clinical information within the original medical image within each of the pattern areas 570 may be de-identified.

In the simplest embodiment, the first pattern area among the pattern areas 570 may be anonymized to have a uniform first signal intensity value, and the second pattern area may be anonymized to have a uniform second signal intensity value. According to an embodiment of the present invention, each of the pattern areas 570 may be anonymized to have a certain sub-pattern within the pattern areas 570. Examples of sub-patterns include sub-patterns such as stripes, hatched patterns, and shading.

Embodiments of the present invention may set the skin area of the human body or the surface area of the human body as a de-identification area in a medical image having three-dimensional anatomical information such as a CT image set or MR image set, and set the de-identification area to a certain size and/or shape. Anonymization/pseudonymization of the de-identification area may be achieved by separating the skin/surface area of the human body into a plurality of pattern areas 570 having a predetermined shape and/or size.

Each of the plurality of pattern areas 570 may be generated to have a randomized signal intensity value.

Each of the plurality of pattern areas 570 may have a signal intensity value generated based on the representative value of the signal intensity values of a de-identification area 540 in each of the plurality of pattern areas 570. Since the signal intensity values of the de-identification area 540 in each of the plurality of pattern areas 570 will have a random distribution based on the arrangement and shape of a skin area, each of the plurality of pattern areas 570 may substantially have a randomized signal intensity value.

The representative value of the signal intensity values may be obtained based on at least one of various statistical means such as the average, median, and/or mode of the signal intensity values. The signal intensity values before anonymization within each of the pattern areas 570 would be different for each of the pattern area 570, and it would not be easy to find any rule. Therefore, if each of the pattern areas 570 may be anonymized using at least one of various statistical means such as the average, median, and/or mode of the signal intensity values before anonymization within each of the pattern areas 570, each of the pattern areas 570 may have a substantially randomized signal intensity value.

Through step S400, the de-identification area 540 in the first medical image may be replaced with the de-identified image 560, thereby generating the second medical image.

The second medical image may include the de-identified image 560 and the finally generated identification area 510. Pattern areas may not be generated in the identification area 510. As a result, the identification area 510 may preserve 3D anatomical structure information without loss.

The first medical image may be a CT image set including a plurality of slices. Step S200 of separating an identification area and a de-identification area, step S300 of generating a de-identified image for the de-identification area, and step S400 of generating a second medical image may be performed for each of the plurality of slices in the CT image set.

The second medical image acquired according to an embodiment of the present invention may be anonymized/pseudonymized without loss of anatomical information inside the human body.

The second medical image acquired according to an embodiment of the present invention may not be visually identified even by volume rendering or 3D reconstruction, so that it can provide a desirable means for de-identification.

In this case, a bone may be located near the skin. Anonymization/pseudonymization may be performed without affecting diagnosis by separately anonymizing only a skin area.

According to an embodiment of the present invention, when the processes of FIGS. 1 to 4 are performed for each of the CT image slices, the size and distribution of the de-identification area 540 may vary for each of the CT image slices.

Pixels/voxels in each of the pattern areas 570 may have a signal value obtained using the representative value of the density or CT intensity values of a skin area, i.e., the de-identification area 540. According to this method, the signal value of each of the pattern areas 570 substantially has a random value, so that it provides the effect of making it difficult to restore the original.

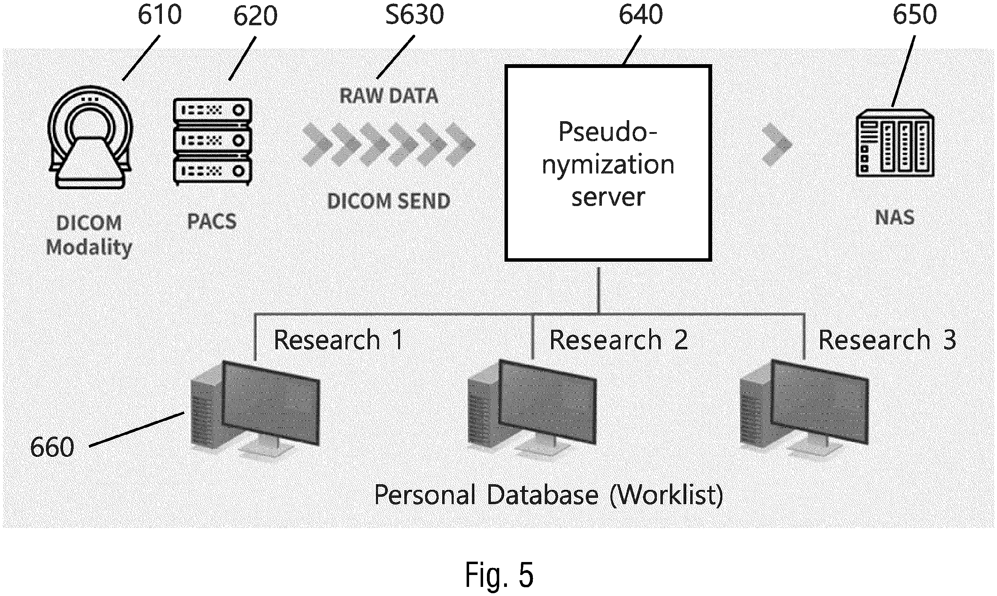

FIG. 5 is a conceptual diagram showing a medical image diagnosis environment in which the results of anonymization/pseudonymization of medical images may be utilized according to an embodiment of the present invention.

Referring to FIG. 5, medical images acquired by a Digital Imaging and Communications in Medicine (DICOM) modality 610 may be sent to a pseudonymization server 640 through the server of a picture archiving and communication system (PACS) 620 in step S630.

The raw medical images may be pseudonymized/anonymized by the pseudonymization server 640, and the pseudonymized/anonymized images may be stored in network attached storage (NAS) 650 or sent to personal databases 660. Although a case where the personal databases 660 are mainly intended for research purposes is shown in FIG. 5, the spirit of the present invention is not limited to this embodiment, and may be utilized for various purposes such as primary diagnosis, incidental findings, and screening for various diseases.

The pseudonymization server 640 may store and manage the raw data obtained by all types of DICOM modalities 610 such as CT and magnetic resonance imaging (MRI), and may pseudonymize the DICOM header values and body surface of patient CT image data.

A general intracorporeal image itself does not allow an individual to be identified using it. In contrast, in the case of a tomographic 3D image in which both the inside and outside of the human body are captured, there are cases where a human body silhouette can be restored using 3D reconstruction.

During this process, a photo of a face or the shape of a specific part of the human body may be exposed. There may be cases where identification is enabled without special additional information.

Accordingly, there is proposed technology in which the pseudonymization server 640 anonymizes the external surface of the body of a patient so that it cannot be identified and internal anatomical information may be preserved without loss.

The pseudonymization server 640 may selectively delete and modify all DICOM values, and may generate de-identified information by assigning IDs in import order. Exceptionally, an administrator account may be assigned the function of searching original information and pseudonymized information.

A second identification area (not shown) in the second medical image may include information that can restore a 3D anatomical structure based on anatomical structure information in the first medical image.

The personal database 660 may restore the 3D anatomical structure based on the information of the second identification area (not shown) in the pseudonymized/anonymized second medical image, and may, based on this, perform not only the primary diagnosis but also incidental findings, screening for various diseases, etc.

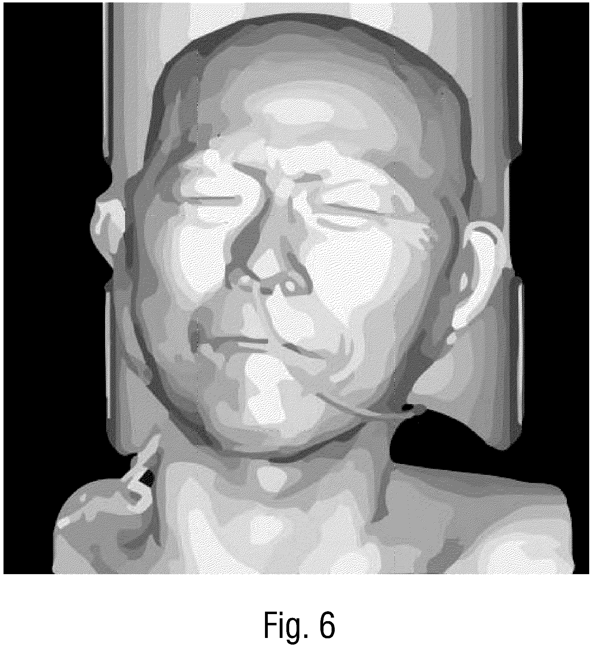

FIGS. 6 to 10 are conceptual diagrams showing a process of anonymizing/pseudonymizing medical images according to embodiments of the present invention.

FIG. 6 is a diagram showing 3D rendering results based on an original medical image.

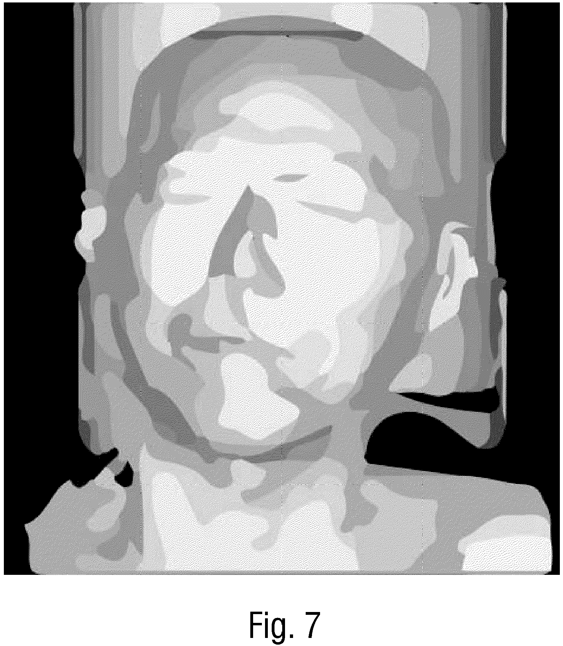

FIG. 7 is a diagram showing the results of 3D rendering performed in the state in which a de-identification area having a thickness of 2 mm is generated.

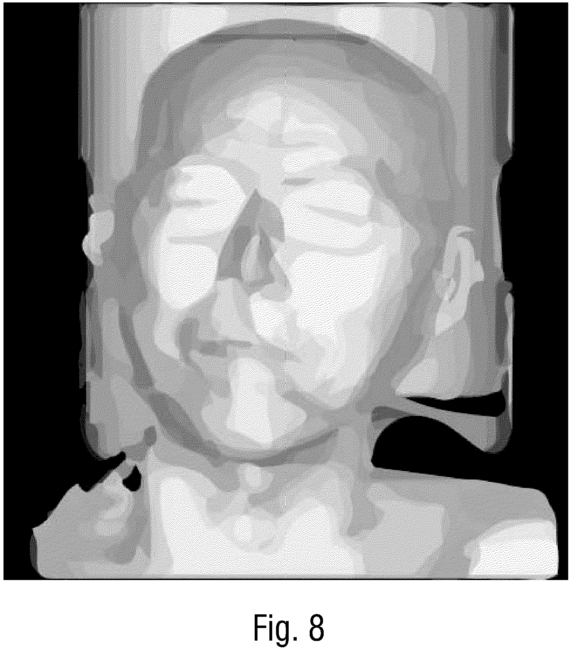

FIG. 8 is a diagram showing the results of 3D rendering performed in the state in which a de-identification area having a thickness of 4 mm is generated.

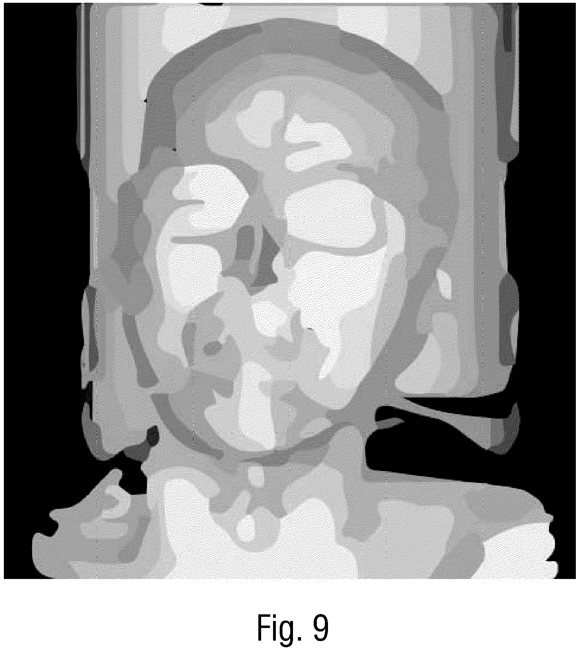

FIG. 9 is a diagram showing the results of 3D rendering performed in the state in which a de-identification area having a thickness of 6 mm is generated.

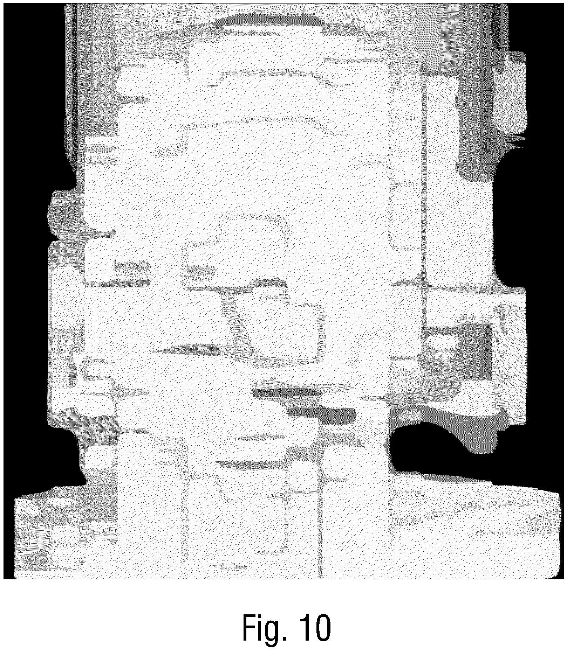

FIG. 10 is a diagram showing the results of 3D rendering performed in the state in which a de-identified image having a 2 mm thickness is generated and then a de-identified image is generated using a mosaic technique.

In the embodiments of FIGS. 7 to 9, this is before a de-identified image is generated using a mosaic technique, so that there is room for the external surface of a patient to be restored to an identifiable level, as in the embodiment of FIG. 6.

The embodiment of FIG. 10 indicates that a de-identified image generated by a mosaic technique is effective for de-identification regardless of the thickness of a de-identification area to be removed.

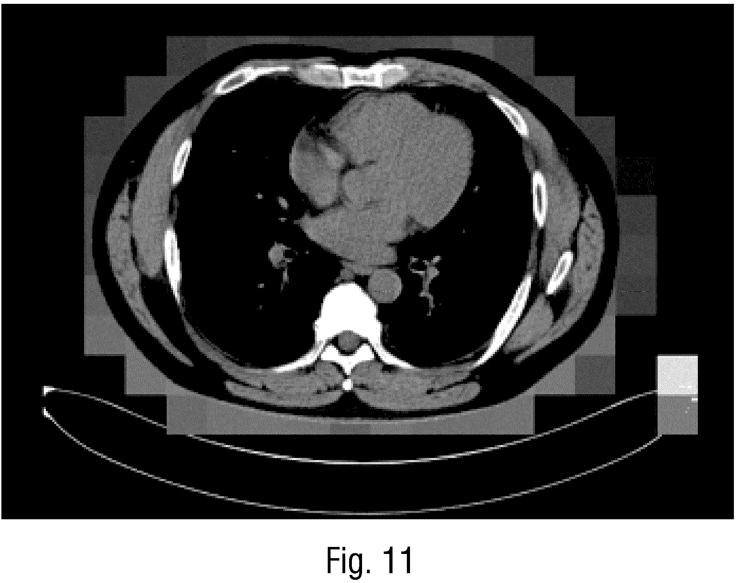

FIG. 11 is a conceptual diagram showing part of a process of anonymizing/pseudonymizing medical images according to an embodiment of the present invention.

As described above, the first medical image may be a CT image set including a plurality of slices. Step S200 of separating an identification area and a de-identification area, step S300 of generating a de-identified image for the de-identification area, and step S400 of generating a second medical image may be performed for each of the plurality of slices in the CT image set.

According to an embodiment of the present invention, the anatomical information of an original medical image may be efficiently reserved during the process of anonymizing/pseudonymizing the medical image.

According to an embodiment of the present invention, there may be provided anonymization/pseudonymization technology that can provide incidental findings based on anatomical information contained in an original medical image.

The present invention may provide anonymization/pseudonymization technology that prevents personal information from being exposed by increasing the level of de-identification of medical images.

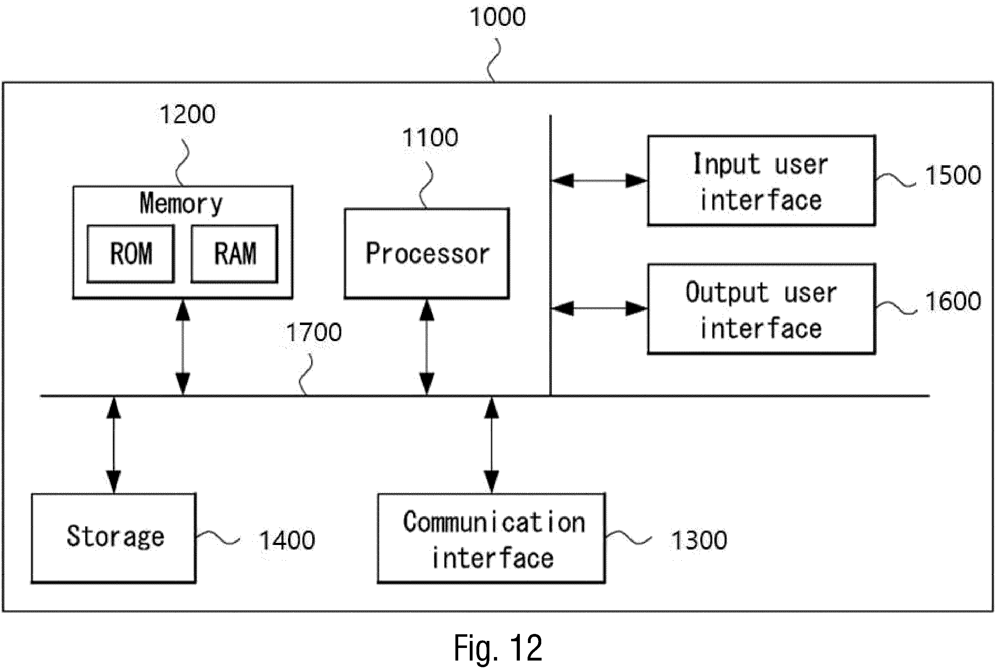

FIG. 12 is a conceptual diagram showing an example of a generalized medical image processing, analysis, visualization, diagnostic assistance, and anonymization/pseudonymization apparatus or computing system capable of performing at least part of the process of FIGS. 1 to 11.

For example, the pseudonymization server 640 of FIG. 5 may be implemented like the computing system of FIG. 12.

At least part of a method for medical image processing, analysis, visualization, diagnostic assistance, and/or anonymization/pseudonymization according to an embodiment of the present invention may be executed by the computing system 1000 of FIG. 12.

Referring to FIG. 12, the computing system 1000 according to an embodiment of the present invention may include a processor 1100, memory 1200, a communication interface 1300, storage 1400, an input interface 1500, an output interface 1600, and a bus 1700.

The computing system 1000 according to an embodiment of the present invention includes at least one processor 1100 and memory 1200 configured to store instructions that instruct the at least one processor 1100 to perform at least one step. At least some steps of the method according to an embodiment of the present invention may be performed in such a manner that the at least one processor 1100 loads instructions from the memory 1200 and executes them.

The processor 1100 may refer to a central processing unit (CPU), a graphics processing unit (GPU), or a dedicated processor on which the methods according to embodiments of the present invention are performed.

Each of the memory 1200 and the storage 1400 may be formed of at least one of a volatile storage medium and a nonvolatile storage medium. For example, the memory 1200 may be formed of at least one of read-only memory (ROM) and random access memory (RAM).

Furthermore, the computing system 1000 may include the communication interface 1300 that performs communication through a wireless network.

Furthermore, the computing system 1000 may further include the storage 1400, the input interface 1500, and the output interface 1600.

Furthermore, the individual components included in the computing system 1000 may be connected by the bus 1700 and communicate with each other.

Examples of the computing system 1000 of the present invention may include a desktop computer, a laptop computer, a notebook, a smartphone, a tablet personal computer (PC), a mobile phone, a smart watch, smart glasses, an e-book reader, a portable multimedia player (PMP), a portable game console, a car navigation device, a digital camera, a digital multimedia broadcasting (DMB) player, a digital audio recorder, a digital audio player, a digital video recorder, a digital video player, and a personal digital assistant (PDA) that are capable of communication.

An apparatus for anonymizing medical images according to an embodiment of the present invention includes memory 1200 including one or more instructions; and a processor 1100 configured to execute the one or more instructions; and the processor 1100, by executing the one or more instructions, may be configured to: acquire or receive a first medical image, separate an identification area containing anatomical structure information and a de-identification area containing a skin area in the first medical image, generate a de-identified image 560 including a plurality of pattern areas 570 with a predetermined size by applying a mosaic technique to the de-identification area, and generate a second medical image by replacing the de-identification area with the de-identified image 570.

In this case, a plurality of pattern areas 570 may include first pattern areas having a first signal intensity value and second pattern areas having a second signal intensity value.

Each of the plurality of pattern areas 570 may be generated to have a randomized signal intensity value.

Each of the plurality of pattern areas 570 may have a signal intensity value generated based on the representative value of the signal intensity values of a de-identification area 540 in each of the plurality of pattern areas 570.

The processor 1100, by executing the one or more instructions, may determine a second de-identification area including at least a portion of the de-identification area and having a thickness based on a user input and generate a plurality of pattern areas 570 having a predetermined size by using the representative value of signal intensity values in the second de-identification area.

The first medical image may be an MR image set or a CT image set including a plurality of slices.

The processor 1100, by executing the one or more instructions, may separate an identification area and a de-identification area, generate a de-identified image for the de-identification area and generate a second medical image for each of the plurality of slices in the CT image set or the MR image set.

The identification area may include information that can restore a 3D anatomical structure based on anatomical structure information in the first medical image.

The operations of the method according to the exemplary embodiment of the present disclosure can be implemented as a computer readable program or code in a computer readable recording medium. The computer readable recording medium may include all kinds of recording apparatus for storing data which can be read by a computer system. Furthermore, the computer readable recording medium may store and execute programs or codes which can be distributed in computer systems connected through a network and read through computers in a distributed manner.

The computer readable recording medium may include a hardware apparatus which is specifically configured to store and execute a program command, such as a ROM, RAM or flash memory. The program command may include not only machine language codes created by a compiler, but also high-level language codes which can be executed by a computer using an interpreter.

Although some aspects of the present disclosure have been described in the context of the apparatus, the aspects may indicate the corresponding descriptions according to the method, and the blocks or apparatus may correspond to the steps of the method or the features of the steps. Similarly, the aspects described in the context of the method may be expressed as the features of the corresponding blocks or items or the corresponding apparatus. Some or all of the steps of the method may be executed by (or using) a hardware apparatus such as a microprocessor, a programmable computer or an electronic circuit. In some embodiments, one or more of the most important steps of the method may be executed by such an apparatus.

In some exemplary embodiments, a programmable logic device such as a field-programmable gate array may be used to perform some or all of functions of the methods described herein. In some exemplary embodiments, the field-programmable gate array may be operated with a microprocessor to perform one of the methods described herein. In general, the methods are preferably performed by a certain hardware device.

The description of the disclosure is merely exemplary in nature and, thus, variations that do not depart from the substance of the disclosure are intended to be within the scope of the disclosure. Such variations are not to be regarded as a departure from the spirit and scope of the disclosure. Thus, it will be understood by those of ordinary skill in the art that various changes in form and details may be made without departing from the spirit and scope as defined by the following claims.

The present invention is a technology developed under the sponsorship of the Seoul Business Agency of the Seoul Metropolitan Government (2022 Bio-Medical Technology Commercialization Support Project; BT220014; Development of Pulmonary Embolism and Pulmonary Hypertension Diagnosis Assistance System through Artificial Intelligence-Based CT Image Automatic Analysis).

## Claims

1. A method of anonymizing medical images, the method comprising:
acquiring or receiving a first medical image;
separating an identification area containing anatomical structure information and a de-identification area containing a skin area in the first medical image;
generating a de-identified image including a plurality of pattern areas with a predetermined size for the de-identification area; and
generating a second medical image by replacing the de-identification area with the de-identified image.

2. The method of claim 1, wherein the plurality of pattern areas comprise first pattern areas having a first signal intensity value and second pattern areas having a second signal intensity value.

3. The method of claim 1, wherein each of the plurality of pattern areas is generated to have a randomized signal intensity value.

4. The method of claim 1, wherein each of the plurality of pattern areas has a signal intensity value generated based on a representative value of signal intensity values of a de-identification area in each of the plurality of pattern areas.

5. The method of claim 1, wherein the generating the de-identified image comprises:
determining a second de-identification area including at least a portion of the de-identification area and having a thickness based on a user input; and
generating the plurality of pattern areas having a predetermined size by using a representative value of signal intensity values in the second de-identification area.

6. The method of claim 1, wherein:
the first medical image is a magnetic resonance (MR) image set or a computed tomography (CT) image set including a plurality of slices; and
the separating, the generating the de-identified image, and the generating the second medical image are performed for each of the plurality of slices in the CT image set or the MR image set.

7. The method of claim 1, wherein the identification area comprises information that can restore a three-dimensional (3D) anatomical structure based on the anatomical structure information in the first medical image.

8. An apparatus for anonymizing medical images, the apparatus comprising:
a memory configured to store one or more instructions; and
a processor configured to execute the one or more instructions;
wherein the processor, by executing the one or more instructions, is configured to:
acquire or receives a first medical image;
separate an identification area containing anatomical structure information and a de-identification area containing a skin area in the first medical image;
generate a de-identified image including a plurality of pattern areas with a predetermined size for the de-identification area; and
generate a second medical image by replacing the de-identification area with the de-identified image.

9. The apparatus of claim 8, wherein the plurality of pattern areas comprise first pattern areas having a first signal intensity value and second pattern areas having a second signal intensity value.

10. The apparatus of claim 8, wherein each of the plurality of pattern areas is generated to have a randomized signal intensity value.

11. The apparatus of claim 8, wherein each of the plurality of pattern areas has a signal intensity value generated based on a representative value of signal intensity values of a de-identification area in each of the plurality of pattern areas.

12. The apparatus of claim 8, wherein the processor, by executing the one or more instructions, is further configured to:
determine a second de-identification area including at least a portion of the de-identification area and having a thickness based on a user input; and
generate the plurality of pattern areas having a predetermined size by using a representative value of signal intensity values in the second de-identification area.

13. The apparatus of claim 8, wherein:
the first medical image is a magnetic resonance (MR) image set or a computed tomography (CT) image set including a plurality of slices; and
the processor, by executing the one or more instructions, is further configured to separate the identification area and the de-identification area, generate the de-identified image for the de-identification area and generate the second medical image for each of the plurality of slices in the CT image set or the MR image set.

14. The apparatus of claim 8, wherein the identification area comprises information that can restore a three-dimensional (3D) anatomical structure based on the anatomical structure information in the first medical image.
